Europäisches Patentamt

(19) European Patent Office

Office européen des brevets

(11) Publication number: **0 372 457**
**A2**

## EUROPEAN PATENT APPLICATION

(21) Application number: **89122335.6**

(22) Date of filing: **04.12.89**

(51) Int. Cl.5: **A61M 5/168**

(30) Priority: **02.12.88 IT 2224288**

(43) Date of publication of application:
**13.06.90 Bulletin 90/24**

(84) Designated Contracting States:
**AT BE CH DE ES FR GB GR IT LI LU NL SE**

(71) Applicant: **SIS-TER S.p.A.**
**Via Crema, 14**
**I-26020 Palazzo Pignano (Cremona)(IT)**

(72) Inventor: **Antonelli, Antonio**
**Via Garibaldi 9 bis**
**26010 MONTE CREMASCO (Cremona)(IT)**

(74) Representative: **Dragotti, Gianfranco et al**
**SAIC BREVETTI s.r.l. Viale Bianca Maria, 15**
**I-20122 Milano(IT)**

(54) Infusion device.

(57) In a flow or infusion device of the type in which the measurement of the number of drops provides an exact value of the infusion flow rate, the insertion of a flow rate measuring device across the dripping chamber ensures the constant delivery of a predetermined amount of solution to be infused.

Fig.1

## Infusion device

The present invention relates to the infusion devices of the type described in the European Patent No.41 182, commercially available under the name of Fleboset-Flowstab.

In these devices, after several years of operation, two problems have been found, namely:

1) When the solution to be administered has a density far different from that of the water casual variations of flow have been observed, which obviously are very important when the hour flow rate injected in the blood circuit must be precisely controlled.

2) Especially at the hospital level often the solutions to be infused are prepared according to a specific recipe of the specialist,whereby a fine and extremely precise adjustement is required, which is hardly obtainable by means of the present scale adjustement of the Flow-stab based on a very limited number of adjustement positions, which are referred to a small number of solutions of standard type, among which no mixtures are foreseen.

As regards the first problem it is already known since some time that if the dripping device is capable of ensuring the delivery of 60 drops per milliliter, the flow of liquid coming out of the infusion device shall remain constant with very different solutions.

The main purpose of the present invention is that of advantageously solving the problems and drawbacks as above shortly mentioned.

Thus the present invention provides an infusion device of the type described in the European patent No. 41 182 (which is herein referred to for a more detailed description) which is characterized in that across the dripping chamber, having a dripping device delivering 60 drops/ml, an electronic device is provided which on the basis of the measurement of the drops, as carried out in a very little time, carries out the integration of the measured data thus determining the value of the actual infusion flow rate (ml/h) and displaying it on an alphanumeric display. The precision of the measurement is consistent with the required one.

The electronic counting devices of the type used in the present invention are well known in the art whereby no detailed description thereof is necessary.

For example reference can be made to photoelectric cell devices which are positioned downstream of the columns for chromatographic separation in the preparation processes using substrates such as modified dextrane and agarose.

The infusion device of the present invention is illustrated in a preferred embodiment thereof, having exemplifying but non limiting purpose, in the accompanying drawings in which, when possible, parts equal or corresponding to those of the EP 41 182 are indicated by the same reference numbers. In the drawings:

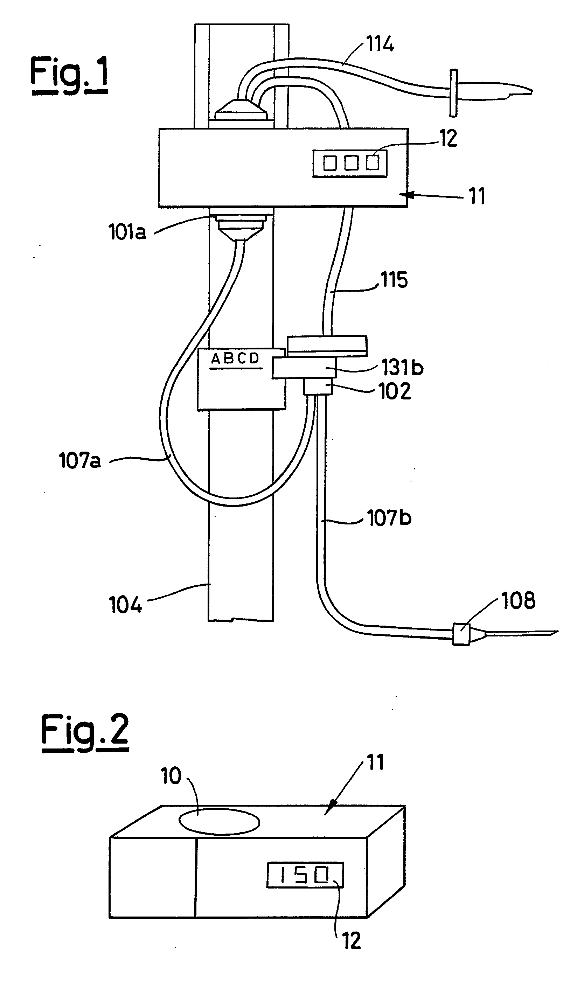

fig.1 is a schematic view of the infusion device and

fig.2 is a schematic view of the electronic device alone. referring to fig. 1, reference 114 indicates the outlet pipe from a bag (either rigid or flexible) containing the solution to be infused, which is downstream connected to the microdripper 101a, contained within the measuring cavity 10 of the electronic counting device 11, having a window 12 for the displaying of alphanumeric data.

This device is of per se well known type and carries out the required measurement in a very small time of the order of 30 seconds.

Through the outlet pipe 107a the solution is conveyed to the delivery device 102, rigidly connected by means of the bracket 131b to the post 104 provided with the positioning scale . The references 107b and 115 respectively indicate the connecting pipes to the infusion needle 108 and to the dripping chamber 101a.

Considering now the operation of the device according to the present, it comprises a dripping chamber provided with a microdripper ensuring a delivery rate of 60 drops/ml. Under these conditions the counting as carried out by the device 11 which is a measurement of the real infusion flow is converted upon being suitably integrated into a numerical value which is seen through the window 12.

At that point it is enough to displace the bracket 131b along the post, controlling at the same time through the display 11 the value which is measured by the counter until the desired delivery flow rate is obtained.

The device according to the present invention furthermore allows for further periodical or random subsequent controls of the infusion pattern whereby unforeseeable variations of the infusion flow rate are prevented, as depending for example from variations of the room temperature.

## Claims

1. Infusion device,of the type described in EP 41 182, characterized in that a device for the electronic measurement of the flow,by counting the delivered drops of liquid,is associated to the dripping chamber, the latter being provided with a microdripper delivering up to 60 drops/ml, said measurement device being provided with means for the alphanumeric display of the measurement carried out.

2.Infusion device according to claim characterized in that saiddripping chamber is surrounded by the measurement chamber of said device for the electronic measurement.

Fig.1

Fig.2